# EUROPEAN PATENT APPLICATION

(11) **EP 4 530 258 A1**
(43) Date of publication of application: **02.04.2025**
(21) Application number: 24192333.3
(22) Date of filing: 01.08.2024
(51) Int. Cl.: C02F 1/32, A61L 2/10, C02F 103/06, C02F 103/08

(54) **FLUID STERILIZATION DEVICE**

(30) Priority: 26.09.2023 JP 2023163447
(71) Applicant: Toshiba Lighting & Technology Corporation, Yokosuka-shi, Kanagawa 237-8510 (JP)
(72) Inventor: OCHI, Takanori, Imabari-shi, Ehime (JP); YASUNAGA, Shigekatsu, Imabari-shi, Ehime (JP)
(74) Representative: Becker, Eberhard

(57) **Abstract**

[Issue] To provide a fluid sterilization device capable of suppressing foreign matters from being attached to a window.

[Solution] A fluid sterilization device (1, 11) according to an embodiment includes: a cylindrical part (2, 12); a light source (5, 15), emitting ultraviolet rays to a fluid flowing through an inside of the cylindrical part via a window (6); a water turbine (7a, 17a), provided inside the cylindrical part; and a removal part (7e, 17c), provided at the water turbine. An end part of the removal part is brought into contact with a surface of the window on a side opposite to a side of the light source.

## Description

### BACKGROUND

### Technical Field

The embodiments of the disclosure relate to a fluid sterilization device.

### Description of Related Art

There have been fluid sterilization devices emitting ultraviolet rays to a fluid, such as water, to exterminate bacteria in the fluid or to inactivate viruses. For example, a fluid sterilization device including a cylindrical part, a window, and a light source has been proposed. A fluid flows through the cylindrical part, the window is provided in the vicinity of an end part of the cylindrical part, and the light source emits ultraviolet rays into the cylindrical part. In such case, a portion of the ultraviolet rays emitted from the light source is directly emitted onto the fluid flowing through the inside of the cylindrical part. In addition, the ultraviolet rays emitted from the light source into the inner side surface of the cylindrical part propagate while being repetitively reflected in the cylindrical part.

Here, such fluid sterilization device may be used at the time of exterminating bacteria in seawater, ground water, etc., or used at the time of inactivating viruses. Meanwhile, seawater, ground water, etc., may contain foreign matters such as sands, dead microorganisms, inorganic salts, etc. Therefore, when the fluid sterilization device is used for such purpose, foreign matters may be attached to the window, and the amount of ultraviolet rays emitted to the fluid may be reduced. When the amount of ultraviolet rays emitted to the fluid is reduced, the effects of exterminating bacteria and inactivating viruses are reduced.

In such case, it takes time and efforts to dissemble the fluid sterilization device to remove the foreign matters attached to the window, and the operational availability of the fluid sterilization device is reduced.
Therefore, it is desired to develop a fluid sterilization device capable of suppressing foreign matters from being attached to the window.

### [Prior Art Document(s)]

### [Patent Document(s)]

[Patent Document 1] Japanese Laid-open No. 2018-069166
[Patent Document 2] Japanese Laid-open No. 2017-051290

### SUMMARY

### [Issues to be solved by the invention]

An issue to be solved by the disclosure is to provide a fluid sterilization device capable of suppressing foreign matters from being attached to the window.

### [Means for solving the issues]

A fluid sterilization device according to an embodiment of the disclosure provides: a cylindrical part; a light source, emitting ultraviolet rays to a fluid flowing through an inside of the cylindrical part via a window; a water turbine, provided inside the cylindrical part; and a removal part, provided at the water turbine. An end part of the removal part is brought into contact with a surface of the window on a side opposite to a side of the light source.

### [Inventive effects]

According to the embodiments of the disclosure, a fluid sterilization device capable of suppressing foreign matters from being attached to the window can be provided.

### BRIEF DESCRIPTION OF THE DRAWINGS

FIG. 1 is a schematic cross-sectional view illustrating a fluid sterilization device according to an embodiment.
FIG. 2 is a schematic cross-sectional view illustrating a fluid sterilization device according to another embodiment.
FIG. 3 is a schematic perspective view illustrating a foreign matter removal part.
FIG. 4 is a schematic perspective view illustrating a water turbine.
FIG. 5 is a schematic perspective view illustrating a support part.
FIG. 6 is a schematic perspective view illustrating a removal part and a biasing part.

### DESCRIPTION OF THE EMBODIMENTS

In the following, the embodiments are described with reference to the drawings. In the respective drawings, like components are referred to with like symbols, and detailed description thereof will be omitted as appropriate. In addition, "sterilization" in the specification not only refers to extermination of bacteria, but also inactivation of viruses. In addition, "sterilization" not only refers to extermination of bacteria, but also covers reduction of bacteria.

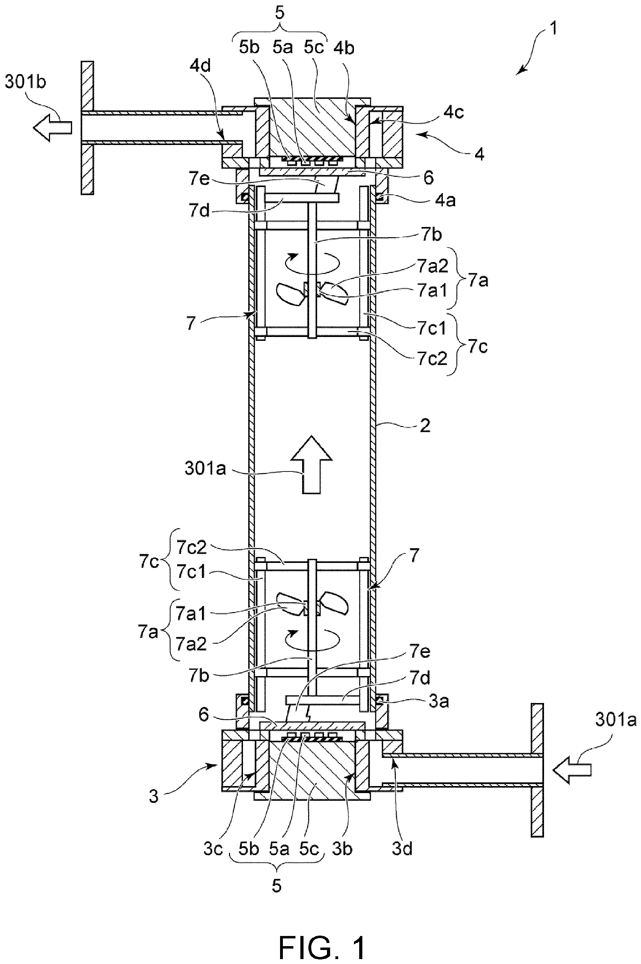

FIG. 1 is a schematic cross-sectional view illustrating a fluid sterilization device 1 according to an embodiment.
As shown in FIG. 1, the fluid sterilization device 1 includes, for example, a cylindrical part 2, a supply head 3, a discharge head 4, a light source 5, a window 6, and a foreign matter removal part 7.

The cylindrical part 2 is in a cylindrical shape, and end parts on both sides are open. The cylindrical part 2 is, for example, a cylindrical tube. Ultraviolet rays are emitted from the light source 5 into the inside of the cylindrical part 2. In this case, when a portion of the emitted ultraviolet rays leaks to the outside via the cylindrical part 2, the processing capability of the fluid sterilization device 1 deteriorates. Therefore, the cylindrical part 2 can be formed from a material that does not allow ultraviolet rays to pass through and has high reflectivity with respect to ultraviolet rays. In addition, the cylindrical part 2 may be formed from a material that is highly resistant against ultraviolet rays and a fluid 301a as the target of sterilization. For example, the cylindrical part 2 can be formed from stainless steel. In such case, if the material of the cylindrical part 2 is stainless steel including 8 wt% or more of nickel (Ni), the erosion resistance against a fluid 301a, such as seawater, which causes erosion easily can be facilitated.

If the cylindrical part 2 contains a material having high reflectivity against ultraviolet rays, the ultraviolet rays emitted into the inner side surface of the cylindrical part 2 are easily reflected toward the fluid 301a. Therefore, the usage efficiency of the ultraviolet rays emitted from the light source 5 can be facilitated. If the usage efficiency of the ultraviolet rays can be facilitated, it is possible to cut down the number of light emitting elements 5a provided in the light source 5. If the number of the light emitting elements 5a is cut down, the light source 5 can be miniaturized, and the cost of the light source 5 as well as the energy consumption thereof can be reduced.

In addition, the inside of the cylindrical part 2 forms the flow path of the fluid 30a as the sterilization target. Therefore, the fluid 301a contacts the inner side surface of the cylindrical part 2. Here, the fluid 301a may be seawater, ground water, etc. Seawater, ground water, etc., may contain foreign matters such as sands, dead microorganisms, inorganic salts, etc. Therefore, when the seawater, ground water, etc., contacts the inner side surface of the cylindrical part 2, the foreign matter may be attached to the inner side surface of the cylindrical part 2. When the foreign matter is attached to the inner side surface of the cylindrical part 2, the reflectivity with respect to ultraviolet rays may decrease. When the reflectivity decreases, the intensity of the reflected light (ultraviolet rays) emitted to the fluid 301a is lowered, so the sterilization effect may be reduced. In such case, if the fluid sterilization device 1 is dissembled to remove the foreign matter attached to the inner side surface of the cylindrical part 2, it is time and effort consuming, and the operational availability of the fluid sterilization device 1 is reduced.

Therefore, a surface roughness (arithmetic mean roughness) Ra of the inner side surface of the cylindrical part 2 may be 50 nanometers (nm) or less, and may also be 3 nm or more and 50 nm or less. In this way, the attachment of the foreign matter to the inner side surface of the cylindrical part 2 can be suppressed, and the reflectivity with respect to the ultraviolet rays can be facilitated. For example, it suffices as long as the surface roughness Ra of the inner side surface of the cylindrical part 2 falls within the range by buffing the inner side surface of the cylindrical part 2.

The supply head 3 is provided at one of the end parts of the cylindrical part 2. A seal member 3a, such as an O-ring, can be provided between the supply head 3 and the end part of the cylindrical part 2. The supply head 3 is in a cylindrically columnar shape, for example, and has a hole 3b penetrating through between the end surface on the side of the cylindrical part 2 and the end surface opposite to the side of the cylindrical part 2. In addition, the supply head 3 has a groove 3c surrounding the hole 3b and open on the end surface on the side of the cylindrical part 2. The opening on the side of the cylindrical part 2 of the groove 3c is connected with the inside of the cylindrical part 2. A supply port 3d is provided on the side surface of the supply head 3. The supply port 3d is connected with the groove 3c.
The supply source of the fluid 301a can be connected with the supply port 3d via a pipe. In addition, a filter, a rectifier, etc., can also be provided at the supply port 3d.

The material of the supply head 3 is not particularly limited, as long as the material is resistant against the fluid 301a and the ultraviolet rays.
The material of the supply head 3 can be a metal, such as stainless steel. As described above, in the case where the fluid 301a is a liquid, such as seawater, that easily causes erosion, the supply head 3 may be formed from stainless steel containing 8 wt% or more of Ni. In this way, even in the case of performing sterilization on seawater, etc., the erosion of the supply head 3 can be suppressed from occurring.

The discharge head 4 is provided at the other end part of the cylindrical part 2. A seal member 4a, such as an O-ring, can be provided between the discharge head 4 and the end part of the cylindrical part 2. The discharge head 4 is in a cylindrically columnar shape, for example, and has a hole 4b penetrating through between the end surface on the side of the cylindrical part 2 and the end surface opposite to the side of the cylindrical part 2. In addition, the discharge head 4 has a groove 4c surrounding the hole 4b and open on the end surface on the side of the cylindrical part 2. The opening on the side of the cylindrical part 2 of the groove 4c is connected with the inside of the cylindrical part 2. A discharge port 4d is provided on the side surface of the discharge head 4. The discharge port 4d is connected with the groove 4c.
A tank accommodating a fluid 301b on which sterilization has been performed can be connected with the discharge port 4d via a pipe. In addition, a filter, a rectifier, etc., can also be provided at the discharge port 4d. The material of the discharge head 4 can be the same as the material of the supply head 3, for example.

In addition, the configuration of the discharge head 4 may be the same as or different from the configuration of the supply head 3. The configuration of the discharge head 4 shown in FIG. 1 is the same as the supply head 3. In the following, as an example, the case where the configuration of the supply head 3 and the configuration of the discharge head 4 are the same is described.

The light source 5 emits, via the window 6, ultraviolet rays to the fluid 301a flowing through the inside of the cylindrical part 2. The light source 5 can be provided in at least one of the supply head 3 and the discharge head 4. The light source 5 shown in FIG. 1 is provided at each of the supply head 3 and the discharge head 4. If the light source 5 is provided at each of the supply head 3 and the discharge head 4, the light amount of the ultraviolet rays emitted onto the fluid 301a flowing through the inside of the cylindrical part 2 can be increased.
Therefore, the sterilization effect can be facilitated. In addition, inside the cylindrical part 2, the region onto which the ultraviolet rays are emitted can be increased. Therefore, even if the length of the cylindrical part 2 in the axial direction is increased, the predetermined sterilization effect can be maintained. Thus, the processing capability can be increased.

If the light source 5 is provided at one of the supply head 3 and the discharge head 4, the configuration of the fluid sterilization device 1 can be simplified, and the manufacturing cost of the fluid sterilization device 1 can be reduced.
Therefore, the number of the light source 5 can be changed as appropriate in accordance with the sterilization effect, the processing capability, the cost, etc., as required.

The light source 5 has, for example, the light emitting element 5a, a substrate 5b, and a base 5c.
The light emitting element 5a is provided on a surface of the substrate 5b on the side of the window 6. The light emitting element 5a emits ultraviolet rays toward the window 6. At least one light emitting element 5a can be provided. The number of the light emitting element 5a can be changed as appropriate in accordance with the processing capability required by the fluid sterilization device 1. In the case where multiple light emitting elements 5a are provided, the light emitting elements 5a are connected in series.

The light emitting element 5a is not particularly limited as long as it is an element that generates ultraviolet rays. The light emitting element 5a can be a light emitting diode, a laser diode, etc., for example.

The peak wavelength of the ultraviolet rays emitted from the light emitting element 5a is not particularly limited, as long as such wavelength exhibits a sterilization effect. However, if the peak wavelength is less than or equal to 300 nm, the ultraviolet rays may be easily absorbed by the DNA or RNA of the bacteria or virus. For example, the light emitting element 5a can be a light emitting diode emitting ultraviolet rays having a peak wavelength of 200 nm to 300 nm.

The substrate 5b is plate-shaped and is provided on an end surface of the base 5c on the side of the cylindrical part 2. A wiring pattern can be provided on the substrate 5b. The material of the substrate 5b may be a material resistant against ultraviolet rays. The material of the substrate 5b can be a ceramic, such as aluminum oxide. The substrate 5b can be a metal core substrate in which the surface of a metal plate is covered by an inorganic material. When the material of the substrate 5b is a ceramic, or the substrate 5b is a metal core substrate, the resistance against ultraviolet rays and high heat-dissipation properties can be attained.

The base 5c can be provided at each of the hole 3b of the supply head 3 and the hole 4b of the discharge head 4. For example, the base 5c can be provided detachably at each of the supply head 3 and the discharge head 4 by using a fastening member, such as a screw. Although the service time of the light emitting element 5a is longer than a discharge lamp, the light emitting efficiency deteriorates as the lighting time increases. In addition, the situation in which the light emitting element 5a has a failure and does not light up is considered. If the bases 5c are detachably provided at the supply head 3 and the discharge head 4, the light emitting element 5a can be replaced easily.

The base 5c has a function of holding the substrate 5b on which the light-emitting element 5a is mounted, as well as a function of discharging the head generated by the light emitting element 5 to the outside. Therefore, the base 5c may be formed from a material with high thermal conductivity. The base 5c can be formed from a metal such as aluminum, copper, stainless steel, etc. In addition, a heat dissipation fin can also be provided at an end surface of the base 5c on the end surface on the side opposite to the side of the cylindrical part 2.

According to the above, the light source 5 including the light emitting element 5a that emits ultraviolet rays has been described. However, the light source 5 is not limited thereto. For example, the light source 5 may also include a discharge lamp, etc., that emits ultraviolet rays. For example, the light source 5 may also be one that includes a low pressure mercury lamp or a barrier discharge lamp. In this case, for example, the light source 5 may be a low-pressure mercury lamp emitting ultraviolet rays having a peak wavelength of 254 nm or a low-pressure mercury lamp emitting ultraviolet rays having peak wavelengths of 185 nm and 254 nm.

The window 6 is in a plate shape and provided between the light source 5 and the inside of the cylindrical part 2. For example, one window 6 can be provided for one light source 5. For example, in the case where the light source 5 is provided at the supply head 3, the window 6 can be provided to block the opening of the hole 3b of the supply head 3. For example, in the case where the light source 5 is provided at the discharge head 4, the window 6 can be provided to block the opening of the hole 4b of the discharge head 4.

The window 6 is opposite to the light source 5. A space can be provided between the window 6 and the light source 5.
The window 6 can be formed from a material that allows ultraviolet rays to pass through and is resistant against ultraviolet rays and the fluid 301a. The window 6, for example, is formed from quartz glass, a fluorine resin that allows ultraviolet rays to pass through, etc.

The ultraviolet rays emitted from the light source 5 are emitted to the fluid 301a flowing through the inside of the cylindrical part 2 via the window 6. A portion of the ultraviolet rays emitted to the inside of the cylindrical part 2 is reflected by the inner side surface of the cylindrical part 2, and the reflected ultraviolet rays are emitted to the fluid 301a. The fluid 301a flowing through the inside of the cylindrical part 2 is thus sterilized by the ultraviolet rays.

In this case, a reflection prevention film can be provided on a surface of the window 6 on the side of the light source 5 (the light emitting element 5a). If the reflection prevention film is provided, the ultraviolet rays emitted from the light emitting element 5a can be prevented from being reflected by the window 6 and having difficulty in emitting ultraviolet rays to the fluid 301a. That is, the usage efficiency of the ultraviolet rays emitted from the light emitting element 5a can be facilitated.

In addition, an anti-fouling film can be provided on a surface of the window 6 on the inner side of the cylindrical part 2. As described above, the fluid 301a may include foreign matters. When a foreign matter is attached to the window 6, it becomes difficult for the ultraviolet rays emitted from the light emitting element 5a to pass through the window 6. With the anti-fouling film being provided, the attachment of a foreign matter to the window 6 can be suppressed.

However, even if an anti-fouling film is provided on the window 6, if there is a great amount of foreign matters, such foreign matters may still be attached to the window 6. In addition, foreign matters may be attached to the window 6 over time. In the case where a foreign matter is attached to the window, if the fluid sterilization device 1 is dissembled to remove the foreign matter attached to the window, such process will take time and efforts. Also, the operational availability of the fluid sterilization device 1 is reduced.

Therefore, the fluid sterilization device 1 is provided with the foreign matter removal part 7.
As shown in FIG. 1, the foreign matter removal part 7 is provided inside the cylindrical part 2. One foreign matter removal part 7 can be provided for one window 6. The foreign matter removal part 7 is opposite to the window 6.
The foreign matter removal part 7, for example, includes a water turbine 7a, a rotation shaft 7b, a support part 7c, a rotation arm 7d, and a removal part 7e.

The water turbine 7a is rotated by using a reaction force when the fluid 301a flows through multiple blades 7a2 of the water turbine 7a. The water turbine 7a is a reaction water turbine. As shown in FIG. 1, the water turbine 7a is a propeller water turbine, which is an example of the reaction water turbine. The water turbine 7a, for example, has a boss 7a1 and multiple blades 7a2 provided on a side surface of the boss 7a1.

The rotation shaft 7b is, for example, in a rod shape, and is provided to be substantially coaxial with the central axis of the cylindrical part 2. The rotation shaft 7b extends through the inside of the cylindrical part 2 in a direction along the central axis of the cylindrical part 2. A through hole extending in the direction along the central axis of the boss 7a1 is provided at the boss 7a1 of the water turbine 7a. The rotation shaft 7b is fixed to the through hole of the boss 7a1. Therefore, the water turbine 7a and the rotation shaft 7b rotate integrally.

The support part 7c has multiple support pillars 7c1 and multiple beams 7c2.
The support pillars 7c1 extend in a direction along the central axis of the cylindrical part 2. The support pillars 7c1 are provided in the vicinity of the inner side surface of the cylindrical part 2, for example. If the support pillars 7c1 are provided in the vicinity of the inner side surface of the cylindrical part 2, the ultraviolet rays emitted from the light source 5 can be suppressed from being shielded by the support pillars 7c1 and decreasing the light amount of the ultraviolet rays emitted to the fluid 301a flowing through the inside of the cylindrical part 2. The end parts of the support pillars 7c1 on the side of the window 6 can be provided on the inner side of the cylindrical part 2, which is the vicinity of the opening of the cylindrical part 2.

The beams 7c2 can be provided side-by-side at predetermined intervals in the direction along the central axis of the cylindrical part 2. The beams 7c2 are in a thin, elongated plate shape, and extend in directions orthogonal to the central axis of the cylindrical part 2. If the beams 7c2 are in a thin, elongated plate shape, the fluid flowing through the inside of the cylindrical part 2 can flow through the sides of the side surfaces of the beams 7c2. Therefore, the flow of the fluid 301a can be suppressed from being interfered.

At the positions of the beams 7c2 on the central axis of the cylindrical part 2, a bearing, such as a ball bearing, is provided. The rotation shaft 7b is provided on the bearing. Therefore, the water turbine 7a and the rotation shaft 7b can be rotatably supported inside the cylindrical part 2.

The rotation arm 7d is provided in the vicinity of the end part of the rotation shaft 7b on the side of the window 6. The rotation arm 7d is in a rod shape, and extends in a direction orthogonal to the central axis of the cylindrical part 2.

The removal part 7e is provided at the water turbine 7a, and an end part of the removal part 7e is brought into contact with a surface of the window 6 on a side opposite to the side of the light source 5. For example, the removal part 7e is provided at the rotation arm 7d The rotation arm 7e is in a plate shape, for example. The end part of the removal part 7e on the side opposite to the side of the rotation shaft 7 is in contact with a surface on the side opposite to the side of the light source 5. The removal part 7e, for example, is formed from a material resistant against the ultraviolet rays and the fluid 301a and elastic to the extent of conforming to the surface of the window 6. The material of the removal part 7e can be a fluorine resin, for example.

The removal part 7e may also be, for example, a brush. In the case where the removal part 7e is a brush, the bristles of the brush are in contact with the surface of the window 6. However, when the brush is used, the bristles of the brush may be mixed with the fluid 301b that has been sterilized. The bristles of the brush mixed into the fluid 301a can be removed by the filter, etc., provided at the discharge port 4d of the discharge head 4.
In such case, if the removal part 7e is a plate-shaped member, the components of the removal part 7e are hardly mixed therein. Therefore, the quality of the fluid 301b having been sterilized can be increased. Also, since it is not required to provide a filter, etc., in the discharge head 4, the configuration of the discharge head 4 can be simplified, or the manufacturing cost can be reduced.

Then, the functions and effects of the foreign matter removal part 7 are described.
The fluid 301a supplied into the cylindrical part 2 via the supply port 3b of the supply head 3 flows through the inside of the cylindrical part 2b toward the discharge head 4. At this time, the fluid 301a is supplied to the blades 7a2 of the water turbine 7a. With the reaction force at the time when the fluid 301a passes through the blades 7a2 of the water turbine 7a, the water turbine 7a rotates. The rotation force of the water turbine 7a is transmitted to the rotation arm 7d via the rotation shaft 7b. Therefore, the removal part 7e provided on the rotation arm 6 is rotationally moved in a direction on the surface of the window 6. The end part of the removal part 7e contacts the surface of the window 6. Therefore, a foreign matter attached to the surface of the window 6 can be peeled off. Also, since the foreign matter in the vicinity of the surface of the window 6 is discharged to the outer side of the window 6, the foreign matter can be prevented from being attached to the surface of the window 6, or the foreign matter having been peeled off can be prevented from being attached again. Therefore, the number of times of dissembling and cleaning the fluid sterilization device 1 can be significantly reduced.
In addition, if the foreign matter removal part 7 is provided, the anti-fouling film of the window 6 can be omitted. Therefore, the manufacturing cost of the window 6 can be reduced. However, if the foreign matter removal part 7 and the anti-fouling film of the window 6 are provided, the foreign matter can be further prevented from being attached to the surface of the foreign matter.

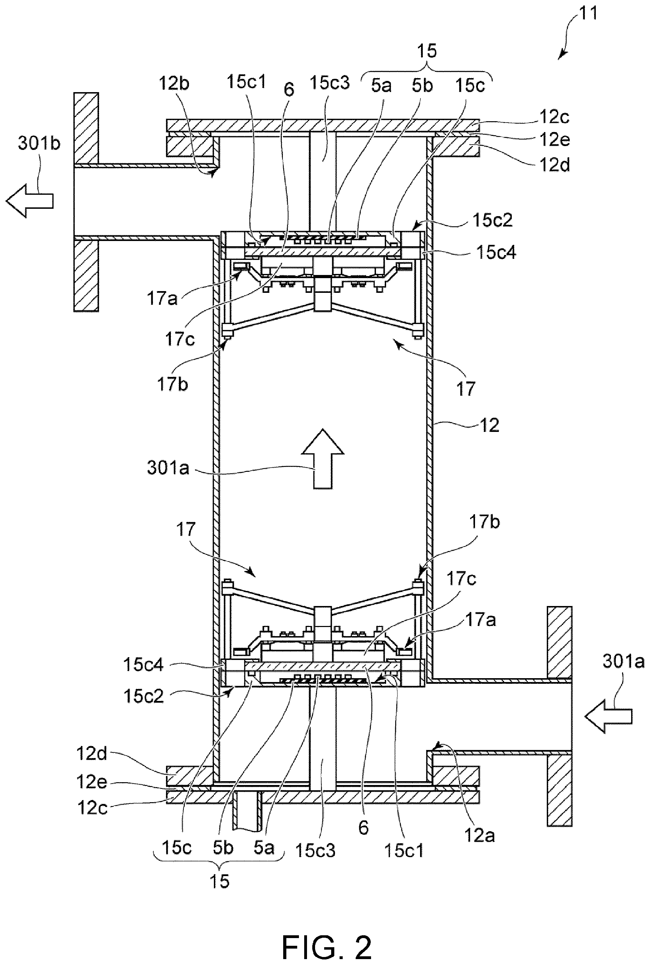

FIG. 2 is a schematic cross-sectional view illustrating a fluid sterilization device 11 according to another embodiment.
As shown in FIG. 2, the fluid sterilization device 11 includes, for example, a cylindrical part 12, a light source 15, the window 6, and a foreign matter removal part 17.

The cylindrical part 12 is in a cylindrical shape, and end parts on both sides are open. The cylindrical part 12 is, for example, a cylindrical tube. The cylindrical part 12 can be the same as the cylindrical part 2. However, a supply port 12a is provided on a side surface of the cylindrical part 12 on the side of an end part. The supply source of the fluid 301a can be connected with the supply port 12a via a pipe. In addition, a filter, a rectifier, etc., can also be provided at the supply port 12a. A discharge port 12b is provided on a side surface of the cylindrical part 12 on the side of the other end part. A tank accommodating the fluid 301b on which sterilization has been performed can be connected with the discharge port 12b via a pipe. In addition, a filter, a rectifier, etc., can also be provided at the discharge port 12b.

The opening of each of the end parts on both sides of the cylindrical part 12 is blocked by a cap 12c. The cap 12c can be detachably provided on a flange 12d provided on the side surface of the cylindrical part 12 by using a fastening member such as a screw. A seal member 12e, such as an O-ring, can be provided between the cap 12c and the flange 12d.

The light source 15 emits, via the window 6, ultraviolet rays to the fluid 301a flowing through the inside of the cylindrical part 12. The light source 15 is provided inside the cylindrical part 12. Like the light source 5, the light source 15 can be provided on at least one of the side of the support port 12a of the cylindrical part 12 and the side of the discharge port 12b of the cylindrical part 12. The light source 15 shown in FIG. 2 is provided on each of the side of the supply port 12a of the cylindrical part 12 and the side of the discharge port 12b of the cylindrical part 12. Like the light source 5, the number of the light source 15 can be changed as appropriate in accordance with the sterilization effect, the processing capability, the cost, etc., as required.

The light source 15 has, for example, the light emitting element 5a, the substrate 5b, and a base 15c.
The base 15c is in a plate shape and has a concave part 15c1 open on an end surface. The substrate 5b on which the light emitting element 5a is mounted is provided on the bottom surface of the concave part 15c1. The opening of the concave part 15c1 is blocked by the window 6 to be liquid-tight. A seal member, such as an O-ring, can be provided between the base 15c and the window 6. The window 6 is sandwiched between the base 15c and a cap 15c4. The cap 15c4 is in a plate shape. In the central portion of the cap 15c4, a hole 15c4a penetrating through in the thickness direction is provided. The window 6 exposes inside the hole 15c4a. In the case of viewing in the direction along the central axis of the cylindrical part 12, the shapes of the base 15 and the cap 15c4 are, for example, circular.

In addition, multiple holes 15c2 penetrating through in the thickness direction are provided on the base 15 and the cap 15c4. The holes 15c2 are arranged side-by-side along the peripheries of the base 15 and the cap 15c4. The central axis of each of the holes 15c2 can be substantially parallel to the central axis of the cylindrical part 12. The holes 15c2 form flow paths of the fluid 301a. In addition, the holes 15c2 also have a function as nozzles that increase the flow velocity of the fluid 301a supplied to blades 17a2 of a water turbine 17a of a foreign matter removal part 17 to be described afterwards.

The base 15c is provided on the cap 12c via a rod-like stand 15c3. In the case where the light source 15 is provided on the side of the supply port 12a of the cylindrical part 12, the light source 15 is supported on the downstream side with respect to the supply port 12a by using a stand 15c3. In the case where the light source 15 is provided on the side of the discharge port 12b of the cylindrical part 12, the light source 15 is supported on the upstream side with respect to the discharge port 12b by using the stand 15c3.

The materials of the base 15c, the stand 15c3, and the cap 15c4 are not particularly limited, as long as the materials are resistant against ultraviolet rays and the fluid 301a. The base 15c, the stand 15c3, and the cap 15c4 can be formed from stainless steel containing 8 wt% or more of Ni.

As shown in FIG. 2, the foreign matter removal part 17 is provided inside the cylindrical part 12. One foreign matter removal part 17 can be provided for one window 6. The foreign matter removal part 17 is opposite to the window 6.

According to the above, the light source 15 includes the light emitting element 5a that emits ultraviolet rays. However, the light source 15 is not limited thereto. For example, like the light source 5, the light source 15 may also include a discharge lamp, etc., that emits ultraviolet rays.

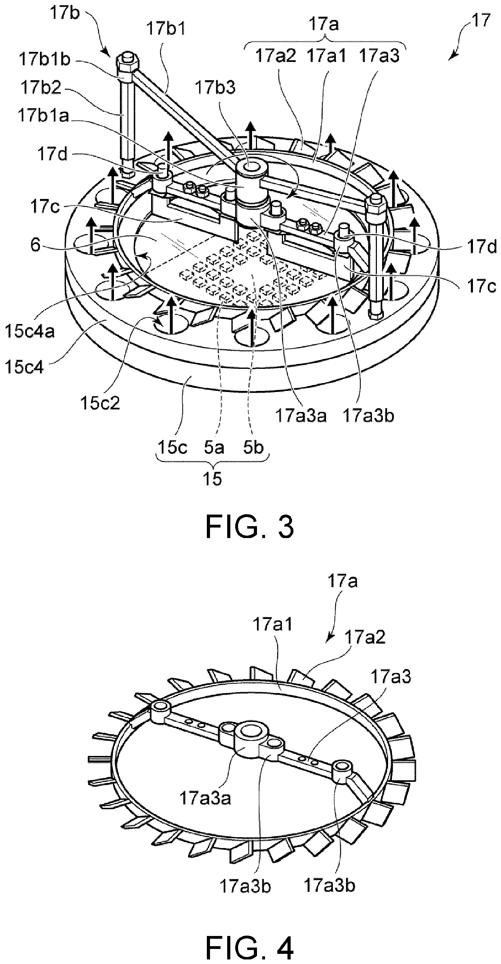

FIG. 3 is a schematic perspective view illustrating the foreign matter removal part 17. As shown in FIG. 3, the foreign matter removal part 17, for example, includes the water turbine 17a, a support part 17b, a removal part 17c, and a biasing part 17d.
FIG. 4 is a schematic perspective view illustrating the water turbine 17a.

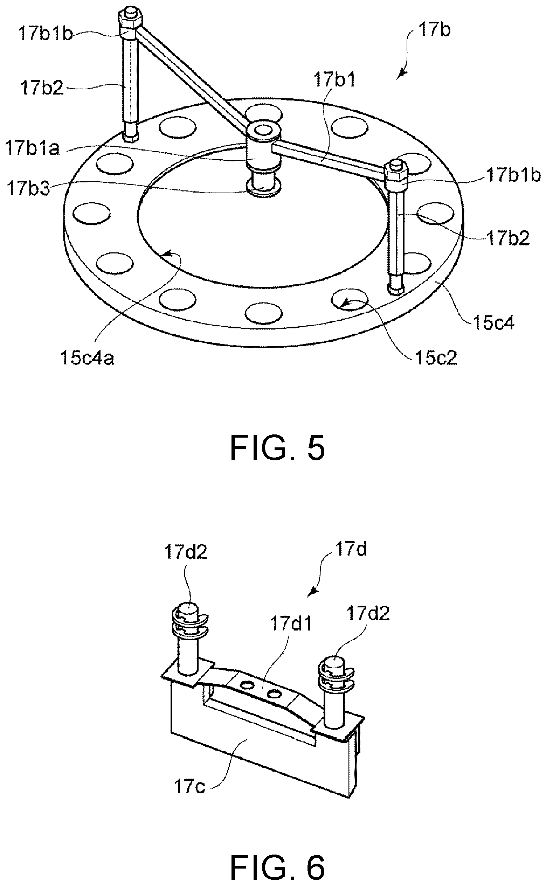

FIG. 5 is a schematic perspective view illustrating the support part 17b.
FIG. 6 is a schematic perspective view illustrating the removal part 17c and the biasing part 17d.

As shown in FIG. 2, the water turbine 17a is provided inside the cylindrical part 12.
As shown in FIGs. 2 and 3, the water turbine 17a is rotatable with the central axis of the cylindrical part 12 as the center. The water turbine 17a can serve as a reaction water turbine that rotates by using the reaction force at the time when the fluid 301a passes through the blades 17a2 of the water turbine 17a.

As shown in FIGs. 3 and 4, the water turbine 17a has, for example, a frame part 17a1, multiple blades 17a2, and a spoke 17a3.
The frame part 17a1 is in a frame shape. In the case of viewing in the direction along the central axis of the cylindrical part 12, the shape of the frame part 17a1 is for example, a ring shape. The frame part 17a1 is located on the inner side with respect to the base 15 and the holes 15c2 provided on the cap 15c4 or on the outer side with respect to the holes 15c2. The frame part 17a1 shown in FIGs. 2 and 3 is located on the inner side with respect to the holes 15c2.

In the case of viewing in the direction along the central axis of the cylindrical part 12, when the water turbine 17a rotates, the blades 17a2 of the water turbine 17a are able to pass through positions overlapped with the holes 15c2. Therefore, in the case where the frame part 17a1 is located on the inner side of the holes 15c2, the blades 17a2 are provided on the outer side surface of the frame part 17a1. In the case where the frame part 17a1 is located on the outer side of the holes 15c2, the blades 17a2 are provided on the inner side surface of the frame part 17a1. The blades 17a2 can be provided at equal intervals on the outer side surface or the inner side surface of the frame part 17a1. The number of the blades 17a2 are not particularly limited. For example, the number of the blades 17a2 can be equal to or more than the number of the holes 15c2.

As shown in FIG. 3, the blades 17a2 are inclined with respect to the central axes of the holes 15c2. The inclination angles of the blades 17a2 can be set as appropriate in accordance with the flow rate or the flow velocity of the fluid 301a. It suffices as long as the inclination angles of the blades 17a2 are determined as appropriate through experimentation or simulation.

Here, as described above, the holes 15c2 form flow paths of the fluid 301a, and serve as nozzles for increasing the flow velocity of the fluid 301a supplied to the blades 17a2. In such case, if the diameter dimension of the hole 15c2 increases, the flow path resistance decreases, and thus it is easy to increase the flow rate.
Meanwhile, if the diameter dimension of the hole 15c2 decreases, the flow velocity of the fluid 301a increases, so the rotation speed of the water turbine 17a (the removal part 17c) can increase and the removal of foreign matters becomes easy.
According to the insight obtained by the inventors, if the flow velocity of the fluid 301a flowing through the hole 15c2 is equal to or more than 1 m/s, the removal of foreign matters becomes easy. The diameter dimension and the number of the holes 15c2 can be determined as appropriate through experimentation or simulation based on the processing flow rate and flow velocity as required.

As shown in FIGs. 3 and 4, the spoke 17a3 is in a rod shape, and extends between two sides on the inner side surface of the frame part 17a1. At least one spoke 17a3 can be provided. However, the more the spokes 17a3, the greater the light amount of the ultraviolet rays shielded by the spokes 17a3. Therefore, the number of the spoke 17a3 may be 1.

The spoke 17a3 has a boss 17a3a and multiple bosses 17a3b. The boss 17a3a is provided at the position of the center of the spoke 17a3. The boss 17a3a has a hole penetrating through in the direction along the central axis of the cylindrical part 12. The boss 17a3a serves as a bearing of the water turbine 17a. A pair of the bosses 17a3b can be provided with respect to one removal part 17c. The boss 17a3b has a hole penetrating through in the direction along the central axis of the cylindrical part 12. Guides 17d2 of the biasing part 17d can be provided at the holes of the pair of bosses 17a3b.

The frame part 17a1, the blades 17a2, and the spoke 17a3 can be formed integrally. The materials of the frame part 17a1, the blades 17a2, and the spoke 17a3 are not particularly limited, as long as the materials are resistant against ultraviolet rays and the fluid 301a. The frame part 17a1, the blades 17a2, and the spoke 17a3 can be formed from a metal, such as an aluminum alloy or stainless steel containing 8 wt% or more of Ni, or resin such as a fluorine resin, etc.

As shown in FIGs. 3 and 5, the support part 17b has, for example, an arm 17b1, a stand 17b2, and a shaft 17b3.
The arm 17b1 is in a rod shape, and extends in a direction intersecting with the central axis of the cylindrical part 12. A boss 17bla can be provided at the central portion of the arm 17b1. A hole penetrating through in the direction along the central axis of the cylindrical part 12 is provided at the boss part 17b1a. The shaft 17b3 is provided at the hole of the boss 17bla. A boss 17blb can be provided at each of the two ends of the arm 17b1. A hole penetrating through in the direction along the central axis of the cylindrical part 12 is provided at the boss part 17blb. The stand 17b2 is provided at the hole of the boss 17blb.

At least one arm 17b1 can be provided. However, like the spoke 17a3, the more the arms 17b1, the greater the light amount of the ultraviolet rays shielded by the arms 17b1. Therefore, the number of the arm 17b1 may be 1.

In addition, the arm 17b1 has a shape bent with the boss 17bla as the center. In such case, in the direction along the central axis of the cylindrical part 12, the distance between the boss 17bla and the light source 15 is shorter than the distance between the boss 17blb and the light source 15. In this way, the distance between the end part side of the arm 17b1 and the light source 15 is increased, so the light amount of the ultraviolet rays emitted to the end part side of the arm 17b1 can be reduced, and the light amount of the ultraviolet rays emitted to the fluid 301a can be suppressed from decreasing. Also, if the distance between the boss 17b1 and the light source decreases, the distance between the boss 17bla and the water turbine 17a decreases, so the posture of the water turbine 17a can be stabilized, and the rotation of the water turbine 17a becomes smooth.

A pair of the stands 17b2 can be provided. An end part of the stand 17b2 is provided at the cap 15c4. The other end part of the stand 17b2 is provided at the hole of the boss 17blb of the arm 17b1.

The shaft 17b3 is provided at the hole of the boss 17bla of the arm 17b1. The shaft 17b3 protrudes from the end part of the boss 17bla on the side of the window 6. The water turbine 17a (the boss 17a3a of the spoke 17a3) is rotatably held at the portion of the shaft 17b3 protruding from the boss 17b1a.

The materials of the stand 17b2 and the shaft 17b3 are not particularly limited as long as the materials are resistant against the fluid 301a and the ultraviolet rays. The materials of the stand 17b2 and the shaft 17b3 can be stainless steel containing 8 wt% or more of Ni.

As shown in FIGs. 3 and 6, the removal part 17c is provided at the water turbine 17a via the biasing part 17d. At least one removal part 17c can be provided. In the foreign matter removal part 17 shown in FIG. 3, a pair of the removal parts 17c are provided. Also, like the removal part 7e, the end part of the removal part 17c on the side opposite to the side of the spoke 17a3 is in contact with a surface on the side opposite to the side of the light source 15. The removal part 17c, for example, is formed from a material resistant against the ultraviolet rays and the fluid 301a and elastic to the extent of conforming to the surface of the window 6.
The material of the removal part 17c can be a fluorine resin, for example.

The biasing part 17d is provided between the water turbine 17a and the removal part 17c. One biasing part 17d can be provided with respect to one removal part 17c. By using elastic force, the biasing part 17d presses the removal part 17c against the surface of the window 6. Therefore, if the biasing part 17d is provided, the sealing property between the end part of the biasing part 17d and the surface of the window 6 can be facilitated, and a foreign matter attached to the surface of the window 6 is peeled off easily.

As shown in FIG. 6, the biasing part 17d has a plate spring 17d1 and a pair of guides 17d2. The central portion of the plate spring 17d1 is provided at the spoke 17a3 of the water turbine 17a. The two ends of the plate spring 17d1 are respectively provided at the guides 17d2. Each of the pair of guides 17d2 is in a rod shape, and is provided at the hole of the boss 17a3b of the spoke 17a3. In such case, the guide 17d2 is movable inside the hole of the boss 17a3b. Therefore, the removal part 17c provided at the biasing part 17d is pressed to conform to the surface of the window 6 by using the elastic force of the plate spring 17d1.

Like the removal part 7e, the removal part 17c may also be, for example, a brush. In the case where the removal part 17c is a brush, the bristles of the brush are in contact with the surface of the window 6. Since the bristles of the brush deform easily, when the removal part 17c is a brush, the biasing part 17d can be omitted. Therefore, the configuration of the foreign matter removal part 17 can be simplified, and the manufacturing cost can be reduced. However, when the brush is used, the bristles of the brush may be mixed with the fluid 301b that has been sterilized. The bristles of the brush mixed into the fluid 301a can be removed by the filter, etc., provided at the discharge port 4d of the discharge head 4. However, if the removal part 17c is a plate-shaped member, the components of the removal part 17c may be hardly mixed therein. Therefore, the quality of the fluid 301b having been sterilized can be increased. Also, since it is not required to provide a filter, etc., in the discharge head 4, the configuration of the discharge head 4 can be simplified, or the manufacturing cost can be reduced.

Then, the functions and effects of the foreign matter removal part 17 are described.
The fluid 301a supplied to the inside of the cylindrical part 12 via the supply port 12a flows through the inside of the cylindrical part 12 toward the discharge head 12b. At this time, the fluid 301a is supplied to the blades 17a2 of the water turbine 17a via the holes 15c2 provided at the cap 15c4 and the base 15c. With the reaction force at the time when the fluid 301a passes through the blades 17a2 of the water turbine 171, the water turbine 17a rotates. The rotation force of the water turbine 17a is transmitted to the removal part 17c via the biasing part 17d.
Therefore, the removal part 17c provided at the water turbine 17a is rotationally moved in a direction on the surface of the window 6. The end part of the removal part 17c is pressed against the surface of the window 6 by the biasing part 71d. Therefore, a foreign matter attached to the surface of the window 6 can be peeled off. Also, since the foreign matter in the vicinity of the surface of the window 6 is discharged to the outer side of the window 6, the foreign matter can be prevented from being attached to the surface of the window 6, or the foreign matter having been peeled off can be prevented from being attached again. Therefore, the number of times of dissembling and cleaning the fluid sterilization device 11 can be significantly reduced.
In addition, if the foreign matter removal part 17 is provided, the anti-fouling film of the window 6 can be omitted. Therefore, the manufacturing cost of the window 6 can be reduced. However, if the foreign matter removal part 17 and the anti-fouling film of the window 6 are provided, the foreign matter can be further prevented from being attached to the surface of the foreign matter.

Although several embodiments of the disclosure have been illustrated above, the embodiments are presented as examples and are not intended to limit the scope of the disclosure. The novel embodiments can be implemented in a variety of other forms, and various omissions, substitutions, modifications, etc., can be made without departing from the spirit of the disclosure. The embodiments and variations thereof are included in the scope and spirit of the disclosure, and are included in the scope of the disclosure and its equivalents described in the claims. Furthermore, the embodiments can be implemented in combination with each other.

The appendices regarding the embodiments are provided in the following.

### (Appendix 1)

A fluid sterilization device, includes:
a cylindrical part;
a light source, emitting ultraviolet rays to a fluid flowing through an inside of the cylindrical part via a window;
a water turbine, provided inside the cylindrical part; and
a removal part, provided at the water turbine. An end part of the removal part is brought into contact with a surface of the window on a side opposite to a side of the light source.

### (Appendix 2)

The fluid sterilization device according to Appendix 1 further includes: a biasing part. The biasing part is provided between the water turbine and the removal part and presses the removal part against the surface of the window by using elastic force.

### (Appendix 3)

In the fluid sterilization device according to Appendix 1, the removal part is a brush, and bristles of the brush contact the surface of the window.

### (Appendix 4)

The fluid sterilization device according to any one of Appendices 1 to 3 further includes a base. The base is provided inside the cylindrical part and having multiple holes penetrating through in a thickness direction.

In a case of viewing in a direction along a central axis of the cylindrical part, when the water turbine rotates, blades of the water turbine pass through positions overlapped with the holes.

### [Reference Signs List]

1: Fluid sterilization device; 2: Cylindrical part; 5: Light source; 5a: Light emitting element; 6: Window; 7: Foreign matter removal part; 7a: water turbine; 7a2: Blade; 7e: Removal part; 11: Fluid sterilization device; 12: Cylindrical part; 15: Light source; 15c: Base; 15c2: Hole; 17: Foreign matter removal part; 17a: water turbine; 17a2: Blade; 17c: Removal part; 17d: Biasing part; 301a: Fluid; 301b: Fluid.

## Claims

1. A fluid sterilization device (1, 11), comprising:
a cylindrical part (2, 12);
a light source (5, 15), emitting ultraviolet rays to a fluid flowing through an inside of the cylindrical part (2, 12) via a window (6);
a water turbine (7a, 17a), provided inside the cylindrical part (2, 12); and
a removal part (7e, 17c), provided at the water turbine (7a, 17a), wherein an end part is brought into contact with a surface of the window (6) on a side opposite to a side of the light source *(5, 15).*

2. The fluid sterilization device (1, 11) as claimed in claim 1, further comprising: a biasing part (17d), provided between the water turbine (7a, 17a) and the removal part (7e, 17c) and pressing the removal part (7e, 17c) against the surface of the window (6) by using elastic force.

3. The fluid sterilization device (1, 11) as claimed in claim 1, wherein the removal part (7e, 17c) is a brush, and
bristles of the brush contact the surface of the window (6).

4. The fluid sterilization device (1, 11) as claimed in claim 1 or 2, further comprising a base (Sc, 15c), provided inside the cylindrical part (2, 12) and having a plurality of holes (15c2) penetrating through in a thickness direction,
wherein, in a case of viewing in a direction along a central axis of the cylindrical part (2, 12), when the water turbine (7a, 17a) rotates, blades (17a2) of the water turbine (7a, 17a) pass through positions overlapped with the holes (15c2).
